# EUROPÄISCHE PATENTSCHRIFT

(11) **EP 0 556 831 B1**
(45) Veröffentlichungstag und Bekanntmachung des Hinweises auf die Patenterteilung: **02.11.1995**
(21) Anmeldenummer: 93102567.0
(22) Anmeldetag: 18.02.1993
(51) Int. Cl.: C07D 311/58

(54) **Verfahren zur Herstellung von 6-Hydroxy-2,5,7,8-tetraalkyl-2-(4-aminophenoxymethyl)chromanen**
Method for the preparation of 6-hydroxy-2,5,7,8-tetraalkyl-2-(4-aminophenoxymethyl)chromans
Procédé pour la préparation des 6-hydroxy-2,5,7,8-tétraalkyl-2-(4-aminophénoxyméthyl)chromanes

(30) Priorität: 21.02.1992 CH 531/92
(43) Veröffentlichungstag der Anmeldung: 25.08.1993
(73) Patentinhaber: LONZA AG, CH-3945 Gampel/Wallis (CH); Sankyo Company Limited, Tokyo (JP)
(72) Erfinder: Heveling, Josef, Dr., Naters (Kanton Wallis) (CH)
(74) Vertreter: Weinhold, Peter, Dr.

(56) Entgegenhaltungen:
- EP-A- 0 207 581
- BULLETIN OF THE CHEMICAL SOCIETY OF JAPAN, Bd. 61, Nr. 9, Juni 1988, TOKYO, JP, Seiten 3283 - 3288 M. SHIBAGAKI, ET AL.: 'The catalytic reduction of aldehydes and ketones with 2-propanol over hydrous zirconium oxide'
- Chemistry Letters 1988, Nr. 10, Seiten 1633-1636

## Beschreibung

Die Erfindung betrifft ein neues Verfahren zur Herstellung von 6-Hydroxy-2,5,7,8-tetraalkyl-2-(4-aminophenoxymethyl)chromanen (Aminochroman) der allgemeinen Formel 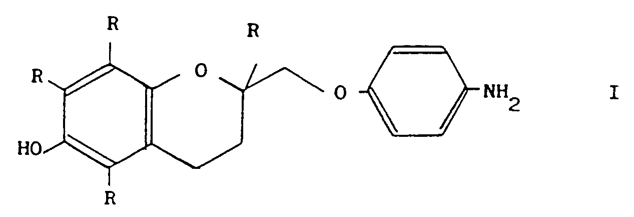
worin R eine Niederalkylgruppe mit 1 bis 4 C-Atomen bedeutet.

Diese Aminochromane der allgemeinen Formel I sind wertvolle Zwischenprodukte zur Herstellung von lipidsenkenden Pharmazeutika (J. Med. Chem. 1989, 32, S. 421)

Aus der EP-A 207 581 ist bekannt, Aminochromane gemäss der allgemeinen Formel I dadurch herzustellen, dass ein Tetraalkyl-2-(4-nitrophenoxymethyl)chroman-4-on zunächst mit Natriumborhydrid in das entsprechende Chroman-4-ol überführt wird, in einer weiteren Stufe das Chroman-4-ol in Gegenwart von p-Toluolsulfonsäure in das Chrom-3-en dehydratisiert wird und im letzten Schritt mit einem Hydrierkatalysator sowohl die Nitrogruppe als auch die Chromen-Doppelbindung zum Endprodukt hydriert wird.

Diese Umsetzung beinhaltet den Nachteil, dass zwischen den einzelnen Reaktionsschritten ein beträchtlicher Aufarbeitungsaufwand anfällt, die eine Umsetzung der Synthese in den technischen Masstab erschwert. Ausserdem ist die Reduktion mit Natriumborhydrid im Vergleich zu katalytischen Reduktionen teuer und aus ökologischer Sicht insofern problematisch, als dass die resultierenden Abwasser borbelastet sind.

Es stellte sich folglich die Aufgabe, eine einfachere Synthese zu entwickeln, die die genannten Nachteile nicht beinhaltet.

Die Aufgabe konnte erfindungsgemäss mit einem Verfahren gemäss Patentanspruch 1 gelöst werden.

Ausgegangen wird von den 6-Hydroxy-2,5,7,8-tetraalkyl-2-(4-nitrophenoxymethyl)chroman-4-onen (Nitrochromanon) der allgemeinen Formel 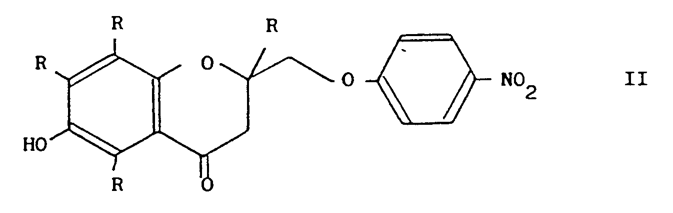
worin R die genannte Bedeutung hat.

Diese können z.B. gemäss EP-PS 139 421 aus Acetylhydrochinonderivaten hergestellt werden.

Vorzugsweise wird vom 6-Hydroxy-2,5,7,8-tetramethyl-2-(4-nitrophenoxymethyl)chroman-4-on (Formel II mit R = CH₃) ausgegangen.

Die Nitrochromanone gemäss Formel II werden erfindungsgemäss in der ersten Stufe in Gegenwart des Katalysatorsystems, amorphes Zirkonoxid/Isopropanol, zu einer Mischung eines 6-Hydroxy-2,5,7,8-tetraalkyl-2-(4-aminophenoxymethyl)chrom-3-en (Aminochromen) der allgemeinen Formel 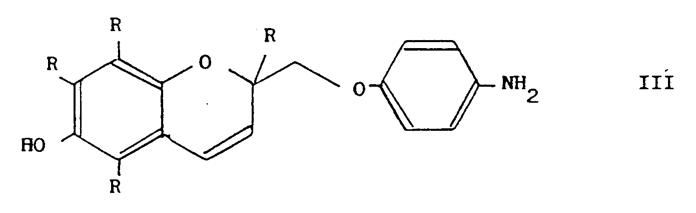
worin R die genannte Bedeutung hat und eines 6-Hydroxy-2,5,7,8-tetraalkyl-2-(4-nitrophenoxymethyl)chrom-3-en (Nitrochromen) der allgemeinen Formel 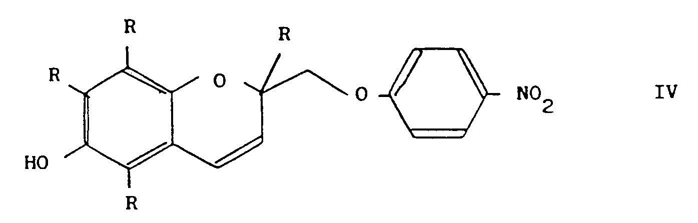
worin R die genannte Bedeutung hat, reduziert.

Das erfindungsgemäss eingesetzte amorphe Zirkonoxid kann auf bekannte Weise durch Fällung aus einer Zirkonylchloridlösung mit Natronlauge, gemäss Shibagaki et al. in Bull. Chem. Soc. Japn. 1988, 61, S. 3283ff (vgl. auch Shibagaki et al., Chem. Letters 1988, 1633), oder mit Ammoniak, gemäss US-PS 5 030 601, und anschliessender Trocknung und Kalzination, hergestellt werden.

Zweckmässig weisen die hergestellten amorphen Zirkonoxide eine spezifische Oberfläche nach BET zwischen 210 m²/g und 265 m²/g auf.
Bevorzugt wird ein amorphes Zirkonoxid eingesetzt, das durch Fällung mit Ammoniak erhalten wird.

Das aus dem Fällungsprozess erhaltene amorphe Zirkonoxid wird vor seinem Einsatz von Vorteil einer Vorbehandlung unterzogen. Dies geschieht zweckmässig durch Behandlung des gefällten und kalziniert amorphen Zirkonoxids in einer mobilen Inertgasatmosphäre bei 150°C bis 300°C über einen Zeitraum von 1 h bis 24 h.

Zweckmässig wird die Umsetzung in der ersten Stufe so durchgeführt, dass das Nitrochromanon gemäss Formel II zusammen mit dem vorbehandelten amorphen Zirkonoxid in Isopropanol, welches als Reaktionspartner und Lösungsmittel fungiert, vorgelegt wird.

Gegebenenfalls kann ein Molekularsieb eingesetzt werden, um aus dem Gleichgewicht Wasser zu entfernen. Damit kann die Reaktionsgeschwindigkeit und die Selektivität günstig beeinflusst werden.

Durch Zusatz einer geringen Menge einer Mineralsäure, wie z.B. Salzsäure, kann die Reaktionsgeschwindigkeit erhöht werden.

Die Umsetzung erfolgt vorzugsweise unter Luftausschluss bei Drucken zwischen zweckmässig 1 bar bis 50 bar, vorzugsweise 20 bar bis 40 bar und einer Temperatur von zweckmässig zwischen 80°C bis 220°C, vorzugsweise 160°C bis 200°C.
Nach einer Reaktionsdauer von in der Regel 3 h bis 10 h ist die Umsetzung beendet.

Aus dem Reaktionsgemisch kann nach Abtrennen des Zirkonoxidkatalysators und Entfernen des Lösungsmittels das Nitrochromen gemäss Formel IV und das Aminochromen gemäss Formel III in Form einer Mischung isoliert werden, wobei der Anteil an Nitrochromen gemäss Formel III üblicherweise überwiegt und in der Regel 75% bis 80% beträgt.

Es ist aber auch möglich, die Reaktionslosung enthaltend die Mischung Nitrochromen der Formel IV und Aminochromen der Formel III nach Abtrennen des Zirkonoxidkatalysators direkt der Folgestufe zuzuführen.

In der Folgestufe wird die genannte Mischung Nitrochromen gemäss Formel IV und Aminochromen gemäss Formel III in Gegenwart eines Hydrierkatalysators mit Wasserstoff zum Endprodukt gemäss Formel I hydriert.

Als Hydrierkatalysator kommen zweckmässig Edelmetallkatalysatoren, wie z.B. Palladium oder Platin aufgebracht auf inerte Trägermaterialien, wie z.B. Kohle oder Aluminiumoxid in Frage.

Vorzugsweise erfolgt die Hydrierung mit einem Palladiumkatalysator, aufgebracht in einer Menge von 0,5% bis 10% auf Kohle als inerten Träger.

Die Hydrierung erfolgt zweckmässig bei einem Wasserstoffdruck von 1 bar bis 20 bar, vorzugsweise 5 bar bis 10 bar, und einer Temperatur von 20°C bis 50°C, vorzugsweise bei Raumtemperatur.

In der Regel ist die Wasserstoffaufnahme nach 0,5 h bis 6 h beendet, worauf das Endprodukt, gemäss Formel I, auf einfache Weise durch Abtrennen des Katalysators und Entfernen des Lösungsmittels isoliert werden kann.

### Beispiele

### A) Herstellung des amorphen Zirkonoxids

Zirkonylchloridoctahydrat (ZrOCl₂·8H₂O) wurde in Wasser gelöst. Die leichte Trübung wurde abfiltriert, und es wurde mit entionisiertem Wasser auf einen Gehalt (ZrO₂) von 50 g/l eingestellt.
Ein technischer Ammoniak (ca. 25%) wurde mit entionisiertem Wasser auf eine Konzentration von 10% verdünnt.

In einem Reaktionsgefäss wurden 2,5 l entionisiertes Wasser vorgelegt. Unter Rühren bei 8000 U/min wurde die Zirkonylchloridlösung und die Ammoniaklösung kontrolliert zudosiert.

Die Dosierrate der ZrO₂-Lösung betrug 50 ml/min. Die Ammoniaklösung wurde so dosiert, dass während der resultierenden Fällung ein pH-Wert von 7,0 ± 0,2 eingehalten werden konnte.

Durch Zusatz von entionisiertem Wasser wurde der Feststoffgehalt der Suspension auf ca. 1% gehalten.
Nach beendeter Fällung wurde der Feststoff durch Filtration abgetrennt. Der Filterkuchen wurde mehrmals mit ammonakialischem Wasser solange gewaschen, bis der Cl⁻-Gehalt auf 0,05% gesunken war.
Der Filterkuchen wurde dann bei 100°C getrocknet, nochmals aufgeschlämmt, filtriert und erneut getrocknet.

Schliesslich wurde das erhaltene ZrO₂-Pulver während 8 h bei 300°C kalziniert. Das erhaltene ZrO₂ war röntgenamorph und hatte eine spezifische Oberfläche nach BET von 240 m²/g.

### Beispiel 1:

### a) Verfahren zur Herstellung von einer Mischung von 6-Hydroxy-2,5,7,8-tetramethyl-2-(4-aminophenoxymethyl)chrom-3-en (Aminochromen, III) und 6-Hydroxy-2,5,7,8-tetramethyl-2-(4-nitrophenoxymethyl)chrom-3-en (Nitrochromen, IV)

2,0 g (5,4 mmol) 6-Hydroxy-2,5,7,8-tetramethyl-2-(4-nitrophenoxymethyl)chroman-4-on (II) wurden mit 5 g amorphem Zirkonoxid (hergestellt gemäss A und vorbehandelt während 2 h bei 200°C unter Argondurchfluss), 100 g Isopropanol (getrocknet mit Molekularsieb 4 Å), 1,0 g Molekularsieb 4 Å (vorbehandelt während 3 h bei 300°C unter Argondurchfluss) und 2,5 ml konzentrierte Salzsäure in einem Autoklaven unter Luftausschluss vorgelegt. Nach mehrmaligem Spülen mit Stickstoff wurde unter Rühren bei 750 U/min bei einem Druck von 10 bar auf eine Temperatur von 190°C erwärmt. Der Druck stieg dabei auf 27 - 31 bar.
Nach 6 bis 7 h wurde die Reaktionsmischung auf Raumtemperatur abgekühlt, das amorphe Zirkonoxid abgetrennt und das Lösungsmittel abgedampft.
Dabei konnten 1,9 g einer Mischung erhalten werden, die 57,5% Nitrochromen (IV) und 16,3% Aminochromen (III) enthielt.

### b) Verfahren zur Herstellung von 6-Hydroxy-2,5,7,8-tetramethyl-2-(4-aminophenoxymethyl)chroman (I)

1,9 g des in Beispiel 1a erhaltenen Gemisches mit einem Gehalt von 73,8% wurden mit 0,4 g eines Palladiumkatalysators auf Kohle (5% Pd/C) in 60 ml Toluol in einem Autoklaven vorgelegt. Nach mehrmaligem Spülen mit Stickstoff und anschliessend mit Wasserstoff wurde bei einem Wasserstoffdruck von 8 bar und bei Raumtemperatur (24 - 27°C) während 3,5 h bis 4 h bei 750 U/min gerührt. Das Reaktionsgemisch wurde anschliessend vom Katalysator befreit. Das Lösungsmittel wurde abgedampft.
Die Rohmischung (1,8 g) enthielt das Titelprodukt in einer Ausbeute von 74,3%.

### Beispiel 2

### Verfahren zur Herstellung von 6-Hydroxy-2,5,7,8-tetramethyl-2-(4-aminophenoxymethyl)chroman (I)

2,0 g (5,4 mmol) 6-Hydroxy-2,5,7,8-tetramethyl-2-(4-nitrophenoxy-methyl)chroman-4-on (II) wurden mit 5 g amorphem Zirkonoxid (hergestellt gemäss A, vorbehandelt während 2 h bei 200°C unter Argondurchfluss), 80 g Isopropanol (getrocknet mit Molekularsieb 4 Å) und 20 g Toluol in einem Autoklaven unter Luftausschluss vorgelegt. Nach mehrmaligem Spülen mit Stickstoff wurde unter Rühren bei 750 U/min bei einem Druck von 10 bar auf eine Temperatur von 190°C erwärmt. Der Druck stieg dabei auf 25 bar.

Nach 5 h wurde die Reaktionsmischung auf Raumtemperatur abgekühlt und das amorphe Zirkonoxid abgetrennt.
Die resultierende Lösung, gemäss GC enthaltend 71,4% einer Mischung von 75,6% Nitrochromen und 24,4% Aminochromen, wurden mit 0,4 g eines Palladiumkatalysators auf Kohle (5% Pd/C) in einem Autoklaven vorgelegt. Nach mehrmaligem Spülen mit Stickstoff und anschliessend mit Wasserstoff wurde bei einem Wasserstoffdruck von 8 bar und bei Raumtemperatur (24 - 27°C) während 1 h bei 750 U/min gerührt. Das Reaktionsgemisch wurde anschliessend vom Katalysator befreit und das Lösungsmittel abgedampft.
Das Rohprodukt (1,8 g) enthielt das Titelprodukt in einer Ausbeute von 75,1%.

## Patentansprüche

1. Verfahren zur Herstellung von 6-Hydroxy-2,5,7,8-tetraalkyl-2-(4-aminophenoxymethyl)chromanen der allgemeinen Formel 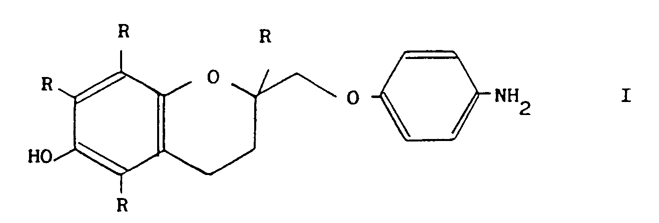 worin R eine Niederalkylgruppe mit 1 bis 4 C-Atomen bedeutet, dadurch gekennzeichnet, dass ein 6-Hydroxy-2,5,7,8-tetraalkyl-2-(4-nitrophenoxymethyl)chroman-4-on der allgemeinen Formel 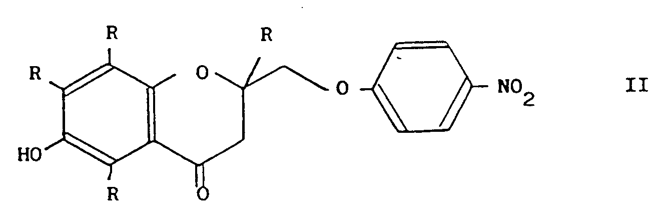 worin R die genannte Bedeutung hat, in Gegenwart des Katalysatorsystems amorphes Zirkonoxid/Isopropanol unter Druck zu einer Mischung eines 6-Hydroxy-2,5,7,8-tetraalkyl-2-(4-aminophenoxymethyl)chrom-3-ens der allgemeinen Formel 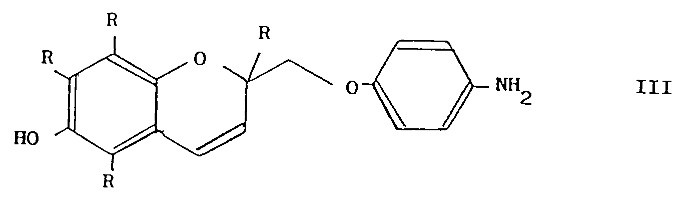 worin R die genannte Bedeutung hat, und eines 6-Hydroxy-2,5,7,8-tetraalkyl-2-(4-nitrophenoxymethyl)chrom-3-ens der allgemeinen Formel 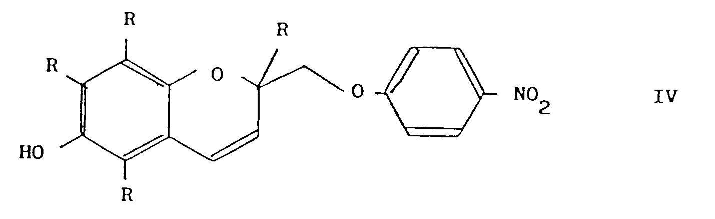 reduziert wird und in der Folgestufe die genannte Mischung in Gegenwart eines Hydrierungskatalysators mit Wasserstoff zum Endprodukt hydriert wird.

2. Verfahren nach Patentanspruch 1, dadurch gekennzeichnet, dass die Reduktion in der ersten Stufe bei einem Druck von 1 bis 50 bar und einer Temperatur zwischen 80°C und 220°C durchgeführt wird.

3. Verfahren nach Patentanspruch 1 oder 2, dadurch gekennzeichnet, dass die Reduktion in der ersten Stufe bei einem Druck von 20 bis 40 bar und einer Temperatur zwischen 160°C und 200°C durchgeführt wird.

4. Verfahren nach einem der Patentansprüche 1 bis 3, dadurch gekennzeichnet, dass für die Reduktion ein amorphes Zirkonoxid eingesetzt wird, das durch ammoniakalische Fällung einer Zirkonylchloridlösung und durch anschliessende Trocknung und Kalzination des gefällten Zirkonoxids hergestellt wird.

5. Verfahren nach Patentanspruch 4, dadurch gekennzeichnet, dass das amorphe Zirkonoxid einer Vorbehandlung in einer mobilen Inertgasatmosphäre bei einer Temperatur zwischen 150°C bis 300°C unterzogen wird.

6. Verfahren nach einem der Patentansprüche 1 bis 5, dadurch gekennzeichnet, dass als Hydrierkatalysator ein Platin- oder Palladiumkatalysator, aufgebracht auf einem inerten Träger, verwendet wird.

7. Verfahren nach Patentanspruch 6, dadurch gekennzeichnet, dass Palladium, aufgebracht in einer Menge von 0,5% bis 10% auf Kohle, verwendet wird.

8. Verfahren nach einem der Patentansprüche 1 bis 7, dadurch gekennzeichnet, dass die Hydrierung bei einem Wasserstoffdruck von 1 bis 20 bar und einer Temperatur von 20°C bis 50°C durchgeführt wird.

## Claims

1. A process for the preparation of 6-hydroxy-2,5,7,8-tetraalkyl-2-(4-aminophenoxymethyl)-chromans of the general formula: 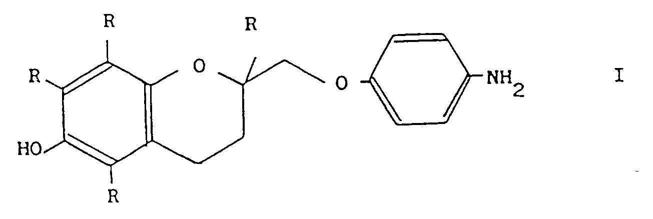 wherein R is a lower alkyl group having 1 to 4 C atoms, characterized in that a 6-hydroxy-2,5,7,8-tetraalkyl-2-(4-nitrophenoxymethyl)-chroman-4-one of the general formula: 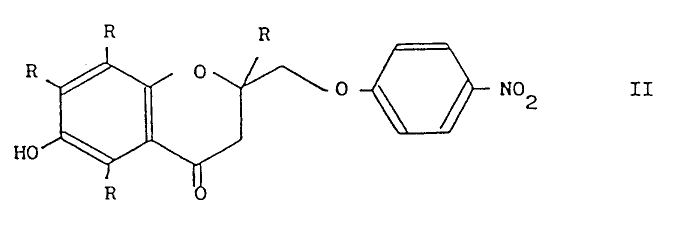 wherein R has the above-stated meaning, is reduced under pressure in the presence of a catalyst system of amorphous zirconium oxide/isopropanol to give a mixture of a 6-hydroxy-2,5,7,8-tetraalkyl-2-(4-aminophenoxymethyl)-chrom-3-ene of the general formula: 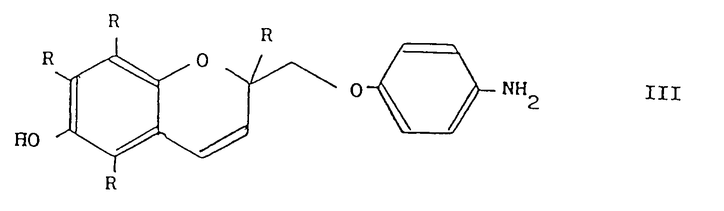 wherein R has the above-stated meaning, and a 6-hydroxy-2,5,7,8-tetraalkyl-2-(4-nitrophenoxymethyl)-chrom-3-ene of the general formula: 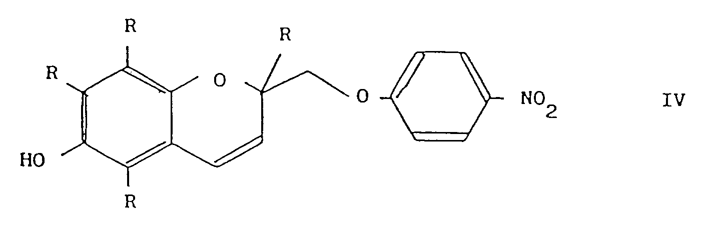 and, in the subsequent step, said mixture is hydrogenated using hydrogen in the presence of a hydrogenation catalyst to give the final product.

2. The process according to claim 1, characterized in that the reduction in the first step is carried out at a pressure of 1 to 50 bar and a temperature of between 80 °C and 220 °C.

3. The process according to claims 1 or 2, characterized in that the reduction in the first step is carried out at a pressure of 20 to 40 bar and a temperature of between 160 °C and 200 °C.

4. The process according to one of the claims 1 to 3, characterized in that, for the reduction, an amorphous zirconium oxide is employed which is prepared by ammoniacal precipitation of a zirconyl chloride solution and subsequent drying and calcination of the precipitated zirconium oxide.

5. The process according to claim 4, characterized in that the amorphous zirconium oxide is subjected to a pretreatment in a mobile inert gas atmosphere at a temperature of between 150 °C to 300 °C.

6. The process according to one of the claims 1 to 5, characterized in that a platinum or palladium catalyst applied to an inert support is used as the hydrogenation catalyst.

7. The process according to claim 6, characterized in that palladium, applied to carbon in an amount from 0.5 % to 10 %, is used.

8. The process according to one of the claims 1 to 7, characterized in that the hydrogenation is carried out at a hydrogen pressure of 1 to 20 bar and a temperature of 20 °C to 50 °C.

## Revendications

1. Procédé pour la préparation des 6-hydroxy-2,5,7,8-tétraalkyl-2-(4-aminophénoxyméthyl)-chromanes de la formule générale 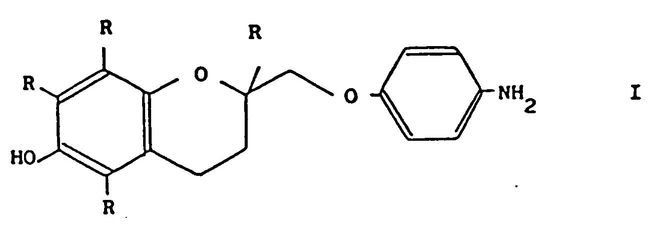 dans laquelle R signifie un groupe alkyle inférieur avec 1 jusqu'à 4 atomes C, caractérisé en ce que l'on réduit une 6-hydroxy-2,5,7,8-tétraalkyl-2-(4-nitrophénoxyméthyl)-chromane-4-one de la formule générale 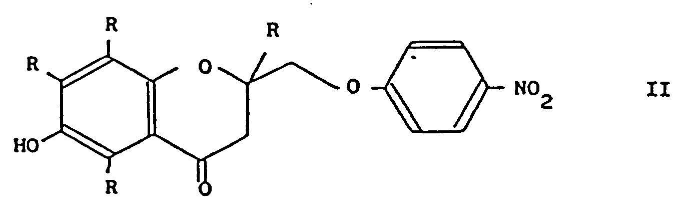 dans laquelle R a la signification citée, en présence du système catalyseur oxyde de zircone amorphe/isopropanol sous pression en un mélange d'un 6-hydroxy-2,5,7,8-tétraalkyl-2-(4-aminophénoxyméthyl)chrom-3-ène de la formule générale 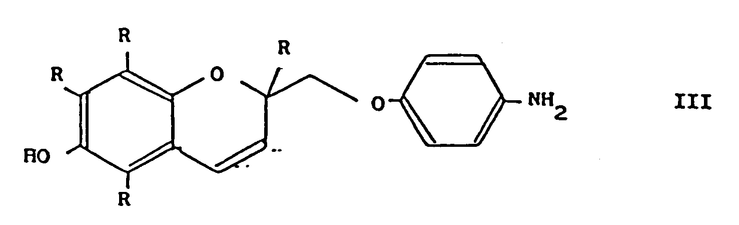 dans laquelle R à la signification citée, et d'un 6-hydroxy-2,5,7,8-tétraalkyl-2-(4-nitrophénoxyméthyl)chrom-3-ène de la formule générale 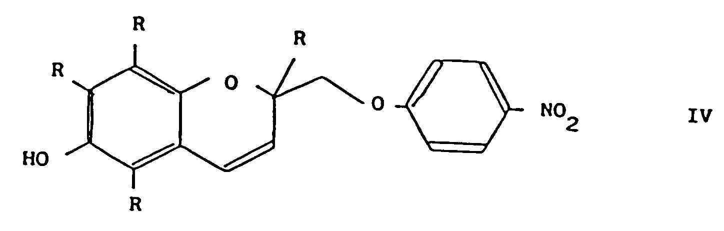 et dans l'étape suivante, on hydrogène le mélange cité d'un catalyseur d'hydrogénation avec de l'hydrogène pour donner le produit final.

2. Procédé selon la revendication 1, caractérisé en ce que dans la première étape la réduction s'effectue à une pression de 1 à 50 bars et à une température entre 80°C et 220°C.

3. Procédé selon la revendication 1 ou 2, caractérisé en ce que dans la première étape la réduction s'effectue à une pression de 20 à 40 bars et à une température entre 160°C et 200°C.

4. Procédé selon l'une des revendications 1 à 3, caractérisé en ce que pour la réduction, on met en oeuvre un oxyde de zircone amorphe qui est préparé par précipitation ammoniacale d'une solution de chlorure de zirconyle et par séchage consécutif et calcination de l'oxyde de zircone précipité.

5. Procédé selon la revendication 4, caractérisé en ce que l'oxyde de zircone amorphe est soumis à un prétraitement dans une atmosphère de gaz inerte mobile à une température entre 150°C et 300°C.

6. Procédé selon l'une des revendications 1 à 5, caractérisé en ce qu'en tant que catalyseur d'hydrogénation on utilise un catalyseur de platine ou de palladium appliqué sur un support inerte.

7. Procédé selon la revendication 6, caractérisé en ce que l'on utilise du palladium appliqué dans une quantité de 0,5 % à 10 % sur du carbone.

8. Procédé selon l'une des revendications 1 à 7, caractérisé en ce que l'hydrogénation est conduite à une pression d'hydrogène de 1 à 20 bars et à une température de 20°C à 50°C.
